# FASCICULE DE BREVET EUROPEEN

(11) **EP 4 165 548 B1**
(45) Date de publication et mention de la délivrance du brevet: **26.11.2025**
(21) Numéro de dépôt: 21737110.3
(22) Date de dépôt: 11.06.2021
(51) Int. Cl.: G06V 20/40

(54) **TRAITEMENT DE FLUX VIDÉO RELATIFS AUX OPÉRATIONS CHIRURGICALES**
VERARBEITUNG VON VIDEOSTRÖMEN IM ZUSAMMENHANG MIT CHIRURGISCHEN OPERATIONEN
PROCESSING OF VIDEO STREAMS RELATED TO SURGICAL OPERATIONS

(30) Priorité: 12.06.2020 FR 2006178
(43) Date de publication de la demande: 19.04.2023
(73) Titulaire: Fondation de Coopération Scientifique, 67000 Strasbourg (FR); Université de Strasbourg, 67000 Strasbourg (FR); Centre National de la Recherche Scientifique (CNRS), 75016 Paris (FR); Università Cattolica del Sacro Cuore, 20123 Milano (IT); Institut De Recherche Contre Les Cancers De L'Appareil Digestif, 67091 Strasbourg (FR)
(72) Inventeur: PADOY, Nicolas, 67000 Strasbourg (FR); MASCAGNI, Pietro, 00187 Roma (IT); DALLEMAGNE, Bernard, 4052 Beaufays (BE)
(74) Mandataire: Plasseraud IP
(86) Numéro de dépôt international: PCT/FR2021/051053
(87) Numéro de publication internationale: WO 2021/250362

(56) Documents cités:
- WO-A1-2019/040705
- WO-A1-2020/023740
- US-A1- 2019 069 957
- US-A1- 2019 362 834

## Description

La présente invention concerne le domaine de l'analyse et du traitement informatique des données relatives à des flux vidéo et notamment aux flux vidéo relatifs aux opérations chirurgicales.

Elle trouve notamment des applications dans des dispositifs de traitement de flux vidéo permettant d'améliorer la sécurité lors des opérations chirurgicales. Par exemple, un dispositif d'assistance pour le chirurgien.

Il existe notamment des protocoles ou des directives pour réduire les complications relatives aux actes chirurgicaux. Toutefois, ces protocoles ou directives ne sont pas nécessairement bien appliqués.

Par exemple, dans le cas d'une ablation de la vésicule biliaire, un protocole permettant d'identifier les organes et leur disposition au cours de l'intervention a été développé, il s'agit de la vision critique de la sécurité (CVS ou « critical view of safety » en anglais).

La CVS concerne trois critères indépendants relatifs à la disposition des organes qui doivent être remplis avant l'ablation. C'est-à-dire que le chirurgien doit disséquer afin de modifier l'anatomie des organes concernés et, basé sur ses observations, doit déterminer dans quelle mesure les trois critères sont remplis avant de passer à l'étape présentant un risque élevé pour le patient.

Malgré des résultats substantiels dans la réduction des complications relatives à ce type d'acte chirurgical, la CVS est peu suivie ou pas nécessairement bien appliquée. De plus, la fiabilité des observations du chirurgien n'est pas constante. Ainsi, même lorsque le protocole CVS est appliqué des erreurs de jugement peuvent advenir.

US 2019/069957 A1 divulgue une méthode de reconnaissance des caractéristiques anatomiques au cours d'une chirurgie robotique.

US 2019/362834 A1 divulgue des techniques permettant de segmenter les vidéos d'une procédure chirurgicale en phases clés et d'extraire des données chirurgicales orientées vers l'apprentissage automatique à partir des segments vidéo pour faciliter l'amélioration des résultats des chirurgies et des compétences des chirurgiens.

WO 2019/040705 A1 divulgue des méthodes d'aide à la décision chirurgicale utilisant un modèle théorique de décision.

WO 2020/023740 A1 divulgue des méthodes pour générer et fournir des conseils chirurgicaux assistés par l'intelligence artificielle.

La présente invention vient améliorer la situation.

Un premier aspect de l'invention concerne un dispositif de traitement d'un flux vidéo relatif à un acte opératoire spécifique, ledit dispositif comprenant :
une interface de réception de flux vidéo ;
un processeur ; et
une mémoire stockant des instructions, de sorte que lorsque ces instructions sont exécutées par le processeur, elles configurent le dispositif pour:
   - réceptionner via l'interface de réception de flux vidéo le flux vidéo comprenant une séquence d'images dont une image à traiter représentant au moins une partie d'un élément anatomique, ladite image à traiter étant formée par des éléments de traitement;
   - déterminer au moyen d'une fonction de traitement si au moins un critère est ou non vérifié sur l'image à traiter, la fonction de traitement étant composé d'une première fonction paramétrée et d'une deuxième fonction paramétrée :
      - des paramètres de la première fonction paramétrée étant obtenus par un algorithme d'apprentissage automatique sur la base d'une image d'une séquence d'images de référence de sorte que le résultat de la première fonction paramétrée appliquée sur l'image permette de déterminer les éléments de traitement de l'image représentatifs de la partie de l'élément anatomique, la séquence d'images de référence étant précédemment enregistrée et relative à l'acte opératoire spécifique, l'image de la séquence d'images de référence représentant au moins une partie d'un élément anatomique,
      - des paramètres de la deuxième fonction paramétrée étant obtenus par un algorithme d'apprentissage automatique sur la base de l'image de la séquence d'images de référence combinée à un résultat de la première fonction paramétrée appliquée sur l'image de la séquence d'images de référence de sorte que le résultat de la deuxième fonction paramétrée appliquée sur l'image combinée à un résultat de la première fonction paramétrée appliquée sur l'image permette de déterminer si l'au moins un critère est ou non vérifié;
   - déterminer un état d'avancement associé à l'image à traiter en fonction de si le critère est vérifié ou non, l'état d'avancement étant représentatif d'un état d'avancement d'une étape opératoire de l'acte opératoire spécifique.

Ainsi, il est possible d'évaluer automatiquement l'état d'avancement d'une étape opératoire, sur la base d'une image du flux vidéo de l'acte opératoire spécifique. Dit autrement, l'analyse de l'image permet de déterminer à quel moment dans le protocole opératoire l'image se situe. Cette analyse se fait en appliquant une fonction de traitement sur l'image, le résultat de cette fonction de traitement indique si un ou plusieurs critères sont ou non vérifiés. En fonction de la validation des critères il est déterminé à quel moment dans le protocole opératoire l'image se situe. Ainsi, il n'est plus nécessaire de s'appuyer sur l'observation du chirurgien pour déterminer l'état d'avancement d'une opération chirurgicale. L'état d'avancement peut ensuite être utilisé pour donner des informations en temps réel au chirurgien. Par exemple, le bon suivi du protocole par le chirurgien, une validation avant de passer à l'étape suivante du protocole, une alerte concernant des organes vitaux et/ou fragiles et/ou peu visibles ou informer d'un élément anatomique avec des caractéristiques anatomiques spécifiques. L'état d'avancement peut également permettre de modifier les paramètres de la vidéo pour s'adapter à l'étape opératoire. Par exemple, il est possible de zoomer sur une zone d'intérêt, d'augmenter le contraste ou encore de modifier la colorimétrie. Il est également possible de stocker avec une meilleure définition les étapes opératoires critiques. Il est également possible de modifier le planning d'utilisation du bloc opératoire en fonction de l'état d'avancement de l'étape opératoire.

Par acte opératoire spécifique, il est entendu un acte médical spécifique, par exemple un acte chirurgical. Autrement dit par acte opératoire spécifique il est entendu un type d'acte médical ou un type d'acte opératoire.

Par flux vidéo, il est entendu une séquence d'images encodées selon un format vidéo (par exemple, MPEG2, H.264/AVC, HEVC, VP8, VP9, AV1). Le flux vidéo peut provenir d'une caméra vidéo, notamment une microcaméra qui peut être introduite dans le corps du patient.

Par éléments de traitement, il est entendu les unités de traitement des images de la séquence, par exemple les pixels. Ces unités de traitement sont propres au dispositif, mais peuvent coïncider avec ceux du format vidéo, par exemple, des macros block (MB) ou encore des « coding tree unit » (CTU).

Par élément anatomique, il est entendu un organe du corps. L'image qui affiche cet élément anatomique le représente.

Plusieurs critères (également appelés critères anatomiques) peuvent être déterminés sur la première image. La détermination de l'état d'avancement peut être indépendante de l'ordre dans lequel ces critères ont été remplis lors du traitement des images précédentes.

Par séquence d'images de référence, il est entendu une séquence d'images filmée préalablement à l'enregistrement de la vidéo dont le flux vidéo est reçu. Il s'agit d'une séquence d'images relative au même type d'acte opératoire que celui du flux vidéo.

Par étape opératoire, il est entendu une étape de l'acte opératoire spécifique. Par état d'avancement de l'étape opératoire, il est entendu un niveau d'avancement ou un niveau de complétude représentatif de l'avancement ou de la complétude de l'étape opératoire, par exemple, un pourcentage de complétion de l'étape.

La fonction de traitement est composée par deux fonctions paramétrées. Par exemple, la première fonction paramétrée est appliquée en premier sur l'image et la deuxième fonction paramétrée est appliquée ensuite sur la combinaison du résultat de la première fonction paramétrée et de cette même image. Autrement dit, la deuxième fonction paramétrée est appliquée sur le résultat de la première fonction paramétrée f₁(I) et l'image I, c'est-à-dire sur le couple (f₁(I) ; I).

La première fonction paramétrée peut être obtenue par un algorithme d'apprentissage automatique entrainé sur une image d'une séquence d'images de référence (et plus généralement sur une collection d'images) afin que la fonction paramétrée renvoie le résultat attendu ou au moins le résultat le plus proche possible. Par exemple, la fonction paramétrée peut être obtenue par optimisation des paramètres d'un réseau de neurones artificiels en appliquant un algorithme d'apprentissage automatique sur l'image (ou la collection d'images). L'algorithme d'apprentissage automatique détermine les paramètres de sorte que ceux-ci optimisent (par exemple, minimise) le résultat d'une fonction de coût calculée pour les valeurs des éléments de traitement (ou simplement sur les éléments de traitement - par exemple les pixels de l'image) de l'image de la séquence d'images de référence (et généralement sur un ensemble important d'images), ledit élément de traitement étant représentatif de la partie de l'élément anatomique. La fonction de coût peut être une distance entre le résultat de la première fonction paramétrée appliquée sur un élément de traitement et le résultat attendu, à savoir, le résultat représentant l'appartenance ou non de l'élément de traitement de l'image à la partie de l'élément anatomique. La première fonction paramétrée permet d'effectuer une segmentation de l'image sur laquelle elle est appliquée en fonction de la partie de l'élément anatomique ou plus généralement des différents éléments anatomiques présent sur l'image.

La première fonction paramétrée est ainsi paramétrée pour permettre de déterminer les éléments de traitement de l'image représentatifs de la partie de l'élément anatomique. Plus précisément, la première fonction paramétrée détermine les éléments de traitement, les identifies, donne des labels ou plus généralement assigne une valeur à chaque éléments de traitement de l'image à traiter en fonction de son appartenance ou non à la partie de l'élément anatomique. La première fonction peut déterminer plusieurs éléments anatomiques différents lorsque les critères à vérifier nécessitent de prendre en compte plusieurs éléments anatomiques. En utilisant un algorithme d'apprentissage automatique, la détermination des paramètres de la première fonction paramétrée est plus précise. De plus, la détermination du groupe d'éléments de traitement est alors moins sensible à l'élément anatomique, c'est-à-dire aux variations de l'élément anatomique d'un patient à un autre. La détermination est donc moins sujette à des erreurs.

La deuxième fonction paramétrée peut être obtenue par un algorithme d'apprentissage automatique entrainé sur l'image de la séquence d'images de référence (et plus généralement sur une collection d'images) combinée avec le résultat de la première fonction paramétrée appliquée sur l'image de la séquence d'images de référence (l'entrainement peut être fait effectivement avec le résultat de la première fonction paramétrée ou directement avec la solution attendue à savoir l'appartenance ou non des éléments de traitement de l'image à la partie de l'élément anatomique) afin que la fonction paramétrée renvoie le résultat attendu ou au moins le résultat le plus proche possible. Par exemple, la fonction paramétrée peut être obtenue par optimisation des paramètres d'un réseau de neurones artificiels en appliquant un algorithme d'apprentissage automatique sur l'image (ou la collection d'images) combinée avec le résultat de la première fonction paramétrée ou la solution attendu.

La combinaison entre l'image de la séquence d'images de référence et le résultat de la première fonction paramétrée appliquée sur l'image de la séquence d'images de référence (ou directement avec la solution attendue à savoir l'appartenance ou non des éléments de traitement de l'image à la partie de l'élément anatomique) est ici nommée simplement combinaison relative à l'image de la séquence d'images de référence ou encore combinaison de référence.

L'algorithme d'apprentissage automatique détermine les paramètres de la deuxième fonction paramétrée de sorte que ceux-ci optimisent (par exemple, minimise) le résultat d'une fonction de coût calculée sur une combinaison de référence (et plus généralement sur un ensemble important de combinaisons relatives à des images de référence). La fonction de coût peut être une distance entre le résultat de la deuxième fonction paramétrée appliquée sur une combinaison de référence et le résultat attendu, à savoir, le résultat représentant si le ou les critères sont ou non vérifiés pour cette combinaison de référence.

Les combinaisons d'une image et du résultat de la première fonction paramétrée appliquée sur cette image ou de la solution attendue peuvent être un n-uplet de matrices dont les cellules représentent les éléments de traitement. Par exemple, trois des n matrices permettent de coder les éléments de traitement (par exemple pixels) en fonction d'un code couleur RVB. Chaque autre matrice du n-uplet représente un élément anatomique différent. Les cellules d'une matrice représentant un élément anatomique on des valeurs (par exemple 1 ou 0) qui dépendent de l'appartenance ou non de l'élément de traitement (correspondant à la cellule) à l'élément anatomique.

Les paramètres de la première et deuxième fonction paramétrée peuvent être stockés sur la mémoire.

En utilisant un algorithme d'apprentissage automatique, la détermination de si le critère est ou non vérifié est plus précise.

Les deux algorithmes d'apprentissage automatique utilisés pour obtenir la première et la deuxième fonction paramétrée sont indépendants l'un de l'autre. Ainsi, il est notamment possible d'effectuer l'apprentissage sur des bases de données distinctes et de mieux maitriser l'apprentissage de chacun des algorithmes, permettant ainsi d'avoir une plus grande efficacité de l'apprentissage tout en requérant moins de puissance de calcul.

Ainsi, cet apprentissage disjoint des deux fonctions paramétrées, plutôt qu'un apprentissage d'une unique fonction qui s'appliquerait sur les images et permettrait d'obtenir directement le résultat de chaque critère, permet de déterminer plus efficacement (notamment avec moins d'erreur ou encore plus de précision) si le critère est ou non vérifié. En effet, en enrichissant l'image avec, pour chaque éléments de traitement de l'image à traiter, une valeur fonction de l'appartenance ou non de l'élément à la partie de l'élément anatomique, il y a moins de risque que des éléments anatomiques soit mal interprété par la deuxième fonction paramétrée et ainsi que les critères soient mal évalués.

L'état d'avancement associé à l'image à traiter peut être déterminé en fonction de la vérification de plusieurs critères (C1, C2, C3), par exemple, chaque combinaison de validation des critères (1, 0, 1), (1, 0, 0), etc. renvoie vers un état d'avancement différent.

Selon un mode de réalisation, lorsque les instructions sont exécutées par le processeur, elles configurent le dispositif pour:
- en fonction de l'état d'avancement déterminé, stocker des images de la séquence d'images comprises dans un intervalle temporel autour de l'image à traiter.

Ainsi, il est possible de stocker une partie du flux vidéo correspondant à des étapes opératoires critiques, ou de stocker cette partie du flux vidéo de manière moins compressée, c'est-à-dire avec une définition plus grande et/ou en conservant plus d'images de cette partie du flux vidéo. Le centre où a eu lieu l'opération peut alors conserver les éléments les plus pertinents de chaque opération.

Selon un mode de réalisation, le nombre d'images stockées est fonction d'une criticité de l'étape opératoire et/ou de l'état d'avancement déterminé.

Ainsi, il est possible d'optimiser l'espace de stockage nécessaire pour stocker la séquence d'image.

Selon un mode de réalisation, il est également prévu un moyen d'affichage et dans lequel lorsque les instructions sont exécutées par le processeur, elles configurent le dispositif pour:
- afficher avec l'image à traité, une information dépendante de l'état d'avancement sur le moyen d'affichage.

Ainsi, le chirurgien ou toute personne relative à l'acte opératoire peut prendre connaissance du niveau d'avancement de l'acte opératoire.

Selon un mode de réalisation lorsque les instructions sont exécutées par le processeur, elles configurent le dispositif pour:
- déterminer un écart avec un protocole opératoire sur la base de l'état d'avancement;
dans lequel l'information dépendante de l'état d'avancement est dépendante de l'écart déterminé.

Ainsi, il est possible d'afficher une information dépendante de l'écart déterminé (par exemple, une alerte) lorsque l'étape opératoire de l'acte opératoire spécifique ne respecte pas un protocole opératoire (par exemple, un protocole opératoire déterminé au début de l'acte opératoire).

Le moyen d'affichage peut se faire notamment sur celui utilisé pour afficher le flux vidéo au chirurgien, par exemple, au moyen d'un écran ou d'un dispositif de vue immersive (par exemple, avec des lunettes de réalité virtuelle).

Par protocole opératoire, il est entendu l'ensemble des tâches opératoires programmées pour réaliser l'acte opératoire spécifique.

L'information dépendante de l'écart est affichée sur le moyen d'affichage, celle-ci peut être insérée dans le flux vidéo affiché au chirurgien, comme précédemment indiqué, par exemple, en réalité augmentée.

L'écart peut être une différence avec le protocole opératoire selon un ou plusieurs indicateurs particuliers, par exemple, en fonction de l'état d'avancement, si le chirurgien réalise une action qui transforme la partie de l'élément anatomique ou une partie d'un autre élément anatomique présent dans les images de la séquence qui ne correspond pas à la transformation attendue selon le protocole opératoire. L'écart peut également correspondre à un décalage temporel entre le temps correspondant à l'état d'avancement dans la séquence d'images et le temps correspondant au même état d'avancement dans la séquence d'images de référence.

Selon un mode de réalisation, l'affichage de l'information dépendante de l'état d'avancement est fonction d'une criticité de l'étape opératoire représentée par l'état d'avancement.

Ainsi, l'information dépendante de l'état d'avancement, par exemple, l'information relative à l'écart pourra être une alerte ou une simple information en fonction de si l'étape opératoire est critique ou non.

Selon un mode de réalisation, l'information dépendante de l'état d'avancement comprend une information indiquant que le critère n'est pas validé.

Ainsi, le chirurgien ou toute personne intervenant dans l'acte opératoire peut en temps réel déterminer les actions ou tâches à mener pour mener à bien l'acte opératoire. Le critère qui n'est pas validé est, par exemple, un critère qui au vu du protocole opératoire aurait dû être validé à ce stade d'avancement.

Selon un mode de réalisation, l'information dépendante du premier état d'avancement comprend une information validant une sous étape, une manœuvre chirurgicale et/ou action chirurgicale d'une étape opératoire ou une information autorisant le démarrage d'une étape opératoire suivante.

Ainsi, il s'agit d'informer le chirurgien du bon déroulement de l'étape opératoire ou de lui indiquer l'étape opératoire suivante, ou encore de valider l'étape opératoire actuelle permettant le passage à l'étape opératoire suivante.

Selon un mode de réalisation lorsque les instructions sont exécutées par le processeur, elles configurent le dispositif pour :
- déterminer un écart entre au moins une valeur d'une caractéristique d'un groupe d'éléments de traitement de l'image représentatifs de la partie de l'élément anatomique et une valeur de référence, la valeur de référence étant une moyenne de la valeur de la caractéristique de groupes d'éléments de traitement représentatifs de la partie de l'élément anatomique et relatif au même état d'avancement que l'état d'avancement;
- déterminer un niveau de risque lié à cet écart ;
dans lequel l'information dépendante de l'état d'avancement comprend le niveau de risque.

Ainsi, il est possible d'alerter le chirurgien lorsqu'un élément anatomique est anormal.

La caractéristique du groupe d'élément peut être, par exemple, la taille du groupe d'éléments de traitement, la forme du groupe d'éléments de traitement, la disposition de ce groupe d'éléments de traitement ou encore la couleur du groupe d'éléments de traitement.

Le niveau de risque peut être défini par des plages de valeurs comprenant la valeur moyenne. Toute valeur de la caractéristique en dehors de la plage de valeur sera considérée comme présentant un niveau de risque.

Plusieurs seuils (ou plages de valeurs) peuvent être définis pour chaque caractéristique surveillée, ainsi, plusieurs niveaux de risque peuvent être déterminés.

La valeur de référence peut être obtenue en faisant la moyenne de valeurs de la caractéristique de groupes d'éléments obtenus à partir d'une pluralité de séquence d'images de référence, les groupes d'éléments étant ceux au même état d'avancement que le premier état d'avancement.

De plus, la détermination de l'écart entre la valeur de la caractéristique du groupe d'éléments de traitement et une valeur de référence est rendue plus efficace car elle utilise le groupe d'éléments de traitement déterminé préalablement, ne requérant ainsi pas de nouvelle détermination.

Selon un mode de réalisation l'information dépendante de l'état d'avancement comprend :
- une zone dans laquelle une action chirurgicale est à réaliser ; et/ou
- une zone dans laquelle il n'y a pas d'action chirurgicale ; et/ou
- une zone dans laquelle se trouve un élément anatomique requérant une attention particulière ; et/ou
- une zone correspondant à des éléments de traitement considérés pour le premier état d'avancement.

Ainsi, il est possible d'afficher sur le moyen d'affichage affichant le flux vidéo au chirurgien une zone évoluant avec l'avancement. Cette zone peut être affichée en surbrillance ou encore par délimitation du contour de cette zone. Ainsi, en fonction de l'état d'avancement, le chirurgien peut être informé de la zone dans lequel il doit opérer et/ou de zones dans lesquelles il n'y a pas à intervenir ou encore d'une zone dans lequel il doit être particulièrement vigilant (par exemple, une zone dans laquelle passe une artère).

La zone peut également correspondre aux éléments de traitement qui ont été pris en compte pour déterminer l'état d'avancement, ainsi le chirurgien ou toute personne intervenant dans l'acte opératoire ou encore tout opérateur du système informatique peut contrôler que le calcul de l'état d'avancement a pris en compte des éléments pertinents. Ce contrôle peut être suivi d'une étape de validation. Ainsi, si le chirurgien ou le tiers opérateur confirme que les éléments pris en compte sont pertinents alors le procédé peut se poursuivre, à l'inverse s'il est infirmé que les éléments pris en compte sont pertinents alors le chirurgien peut être informé que la fiabilité de la détermination de l'état d'avancement est faible. Déterminer si les éléments pris en compte sont pertinents, c'est-à-dire, identifier si le groupe d'éléments de traitement déterminé est pertinent pour déterminer l'état d'avancement peut être réalisé de manière automatique (par exemple, en comparant avec un protocole opératoire) ou de manière manuelle avec les connaissances du chirurgien.

Selon un mode de réalisation l'information dépendante de l'état d'avancement comprend des images de la séquence d'images de référence en commençant par l'image correspondant à l'état d'avancement.

Ainsi, le chirurgien peut voir le déroulement d'un acte opératoire similaire. De plus, la partie de la séquence d'images qui est affichée sur le moyen d'affichage correspond à la même étape opératoire que celle pour laquelle l'état d'avancement a été déterminé, c'est-à-dire l'étape en cours de déroulement.

Selon un autre aspect, il est proposé un programme informatique comportant des instructions qui lorsque celles-ci sont exécutées par un processeur mettent en œuvre un procédé de traitement d'un flux vidéo relatif à un acte opératoire spécifique comprenant :
- la réception via l'interface de réception de flux vidéo du flux vidéo comprenant une séquence d'images dont une image à traiter représentant au moins une partie d'un élément anatomique, ladite image à traiter étant formée par des éléments de traitement;
- la détermination au moyen d'une fonction de traitement de si un critère est ou non vérifié sur l'image à traiter, la fonction de traitement étant composé d'une première fonction paramétrée et d'une deuxième fonction paramétrée :
   - des paramètres de la première fonction paramétrée étant obtenus par un algorithme d'apprentissage automatique sur la base d'une image d'une séquence d'images de référence de sorte que le résultat de la première fonction paramétrée appliquée sur l'image permette de déterminer les éléments de traitement de l'image représentatifs de la partie de l'élément anatomique, la séquence d'images de référence étant précédemment enregistrée et relative à l'acte opératoire spécifique, l'image de la séquence d'images de référence représentant au moins une partie d'un élément anatomique,
   - des paramètres de la deuxième fonction paramétrée étant obtenus par un algorithme d'apprentissage automatique sur la base de l'image de la séquence d'images de référence combinée à un résultat de la première fonction paramétrée appliquée sur l'image de la séquence d'images de référence de sorte que le résultat de la deuxième fonction paramétrée appliquée sur l'image combinée à un résultat de la première fonction paramétrée appliquée sur l'image permette de déterminer si le critère est ou non vérifié;
- la détermination d'un état d'avancement associé à l'image à traité en fonction de si le critère est vérifié ou non, l'état d'avancement étant représentatif d'un état d'avancement d'une étape opératoire de l'acte opératoire spécifique.

Selon un autre aspect de l'invention, il est proposé un support d'enregistrement non transitoire, lisible par un ordinateur, sur lequel est enregistré un tel programme.

Selon un autre aspect de l'invention, il est proposé un système comprenant le dispositif de traitement de flux vidéo, une caméra comprenant un endoscope reliée à l'interface de réception de flux vidéo.

D'autres caractéristiques, détails et avantages de l'invention apparaîtront à la lecture de la description détaillée ci-après, et à l'analyse des dessins annexés, sur lesquels :
[Fig. 1] la figure 1 illustre le dispositif selon un mode de réalisation particulier ;
[Fig. 2] la figure 2 illustre un ordinogramme représentant une mise en œuvre du dispositif selon un mode de réalisation particulier.

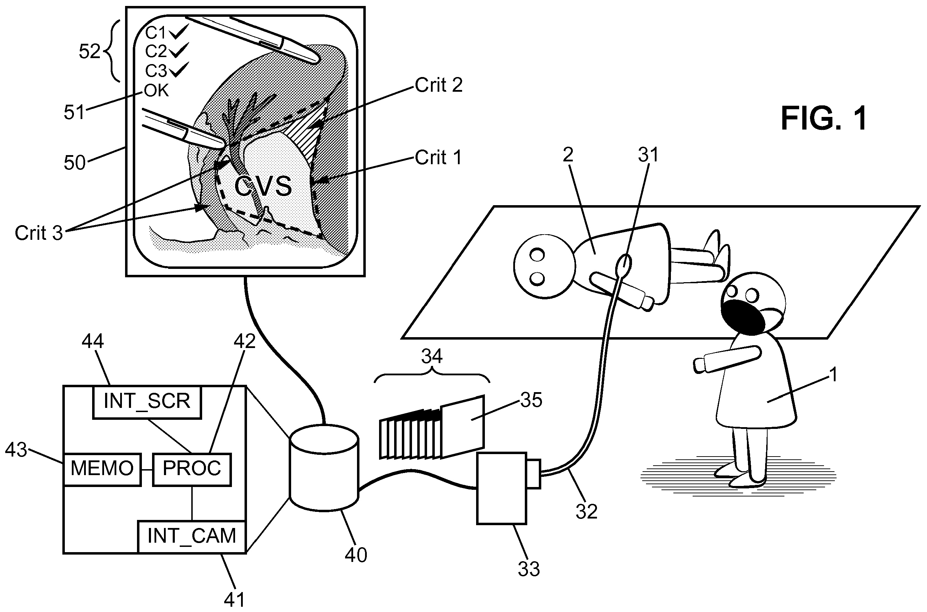

L'exemple de la figure 1 décrit une salle de bloc opératoire où un chirurgien 1 pratique un acte opératoire spécifique, par exemple, une cholecystectomies (ablation de la vésicule biliaire) d'un patient 2.

L'acte opératoire se déroule avec une caméra chirurgicale, comprenant une lentille 31, une fibre optique 32 et un module d'encodage 33 permettant de convertir le signal lumineux provenant de la lentille 31 et transféré par la fibre optique 32 en un signal numérique, à savoir un flux vidéo. La caméra chirurgicale représentée ici est une caméra laparoscopique. Tout autre dispositif de caméra chirurgicale peut être employé avec l'invention.

Le flux vidéo comprend une séquence d'images 34 encodée dans un format vidéo, par exemple, un format MPEG. Ce flux vidéo est transmis au dispositif de traitement du flux vidéo 40.

Le dispositif de traitement de flux vidéo 40 comprend un module d'interface avec la caméra chirurgicale (INT_CAM) 41, un processeur (PROC) 42, une mémoire (MEMO) 43 et un module d'interface avec un moyen d'affichage (INT_SCR) 44.

Le processeur 42 est configuré pour déterminer au moyen de la fonction de traitement F si les images 35 traitées vérifient ou non le ou les critères (Crit 1, Crit 2, Crit 3) et pour déterminer des états d'avancement associés aux images 35 traitées.

Le processeur 42 peut être configuré pour déterminer les paramètres de la première et deuxième fonction paramétrée au moyen de séquences d'images de référence qui ont été enregistrées précédemment à l'acte opératoire en cours et peuvent être stockées sur le dispositif de traitement de flux vidéo 40 ou dans une base de données distante (non représentée ici).

Le processeur 42 est configuré pour stocker des images de la séquence qui sont critiques.

Le processeur 42 est configuré pour déterminer un niveau de risque relatif à un élément anatomique présentant une anormalité.

Le processeur 42 est configuré pour déterminer des écarts entre des situations de l'acte opératoire correspondant aux images 35 et un protocole opératoire.

Le processeur 42 est également configuré pour contrôler le module d'interface avec la caméra chirurgicale (INT_CAM) 41 afin de pouvoir recevoir le flux vidéo provenant du module d'encodage 33 de la caméra chirurgicale.

Le processeur 42 est également configuré pour contrôler le module d'interface avec le moyen d'affichage (INT_SCR) 44 afin de pouvoir afficher au chirurgien 1 le flux vidéo accompagné de différentes informations.

La mémoire 43 comprend une mémoire non volatile sur laquelle est stocké le programme informatique et une mémoire volatile sur laquelle sont stockés les paramètres des fonctions paramétrées (à savoir les paramètres de la fonction f₁ (première fonction paramétrée) qui réalise la segmentation des images à traiter 35 et de la fonction test des critères f₂ (deuxième fonction paramétrée)), les images du flux vidéo, les informations à afficher sur le moyen d'affichage...

Une fois la séquence d'images 34 traitée, le dispositif de traitement du flux vidéo 40 peut transmettre des informations à afficher sur un moyen d'affichage 50, via le module d'interface avec le moyen d'affichage (INT_SCR) 44.

Le moyen d'affichage 50 de la figure 1 est un écran dans la salle du bloc opératoire, mais tout autre moyen d'affichage utilisé dans le cadre des opérations chirurgicales peut être employé, par exemple des lunettes de réalité augmentée.

Le dispositif de traitement du flux vidéo 40 et le moyen d'affichage peuvent ne pas être situés dans la salle du bloc opératoire ou même dans les bâtiments dans lesquels se situe la salle du bloc opératoire. Par exemple, le dispositif de traitement du flux vidéo 40 peut être dans un centre de données hébergent des services relatifs aux technologies de l'information (IT), et le moyen d'affichage peut être dans la salle ou se trouve le chirurgien 1, salle qui peut ne pas être la salle du bloc opératoire où se trouve le patient 2 dans le cas d'une opération chirurgicale à distance.

Le stockage des images de la séquence qui sont critiques peut être réalisé sur une base de données de l'hôpital ou encore dans un centre de données extérieur.

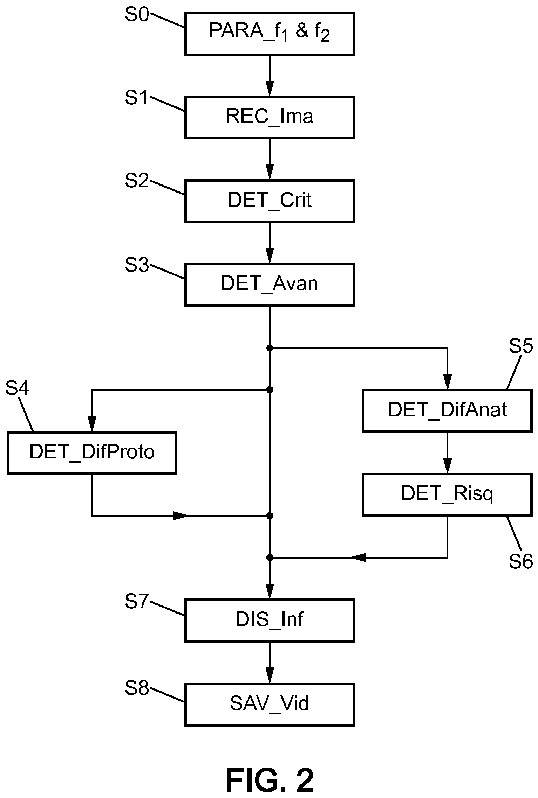

La figure 2 représente un organigramme d'un procédé selon un mode de réalisation particulier.

A l'étape S0, le dispositif est paramétré pour un certain type d'acte opératoire, par exemple dans le cas décrit ici une cholécystectomie ou ablation de la vésicule biliaire. Pour cela une fonction de traitement F est déterminée et stockées sur la mémoire 43 du dispositif 40.

La fonction de traitement F est composée de deux fonctions, une première fonction paramétrée f₁ et une deuxième fonction paramétrée f₂. La première fonction paramétrée f₁ permet la segmentation des images 35 à traiter, la deuxième fonction paramétrée f₂ permet de tester si les critères C1, C2 et C3 sont vérifiés sur l'image I. La fonction F peut par exemple associer à une image I à traiter 35 le triplet (c1, c2, c3) obtenu par F(I) = f₂(I, f₁(I)). Le couple (I, f₁(I)) peut être un n-uplet (M1,.., Mn) de matrices dont les cellules représentent les éléments de traitement. Les valeurs des cellules de la matrice M1 représentent le niveau de couleur rouge dans le code couleur RVB, chaque cellule de la matrice M1 représentant un élément de traitement (par exemple un pixel) de l'image I à traiter 35. Les valeurs des cellules de la matrice M2 représentent le niveau de couleur vert dans le code couleur RVB, chaque cellule de la matrice M2 représentant un élément de traitement (par exemple un pixel) de l'image I à traiter 35. Les valeurs des cellules de la matrice M3 représentent le niveau de couleur bleue dans le code couleur RVB, chaque cellule de la matrice M3 représentant un élément de traitement (par exemple un pixel) de l'image I à traiter 35. Chacune des matrices M4 à Mn correspond à un élément anatomique différent. Chaque cellule de la matrice Mi (pour i allant de 4 à n) représente un élément de traitement (par exemple un pixel) de l'image I à traiter 35, la valeur (par exemple, 0 ou 1) de la cellule code le fait que l'élément de traitement représente ou non une partie de l'élément anatomique correspondant à la matrice Mi. f₁(I) correspond au matrices M4 à Mn, par exemple f₁(I) = (M4,..., Mn). L'image I à traiter 35 est représentée par les matrices M1, M2 et M3. D'autres représentations du couple (I, f₁(I)) sont possibles et n'affectent pas l'application de l'invention. Par exemple, M1, M2, et M3 peuvent être remplacées par une unique matrice dont les cellules représentent directement le code RVB. Ainsi, f₁ associe à l'image I à traiter 35 les matrices (M4,..., Mn). Par exemple, si la segmentation de l'image s'opère sur la base de sept éléments anatomiques, alors f₁ associe à l'image I à traiter 35 les matrices (M4,..., M10). Chacune des matrices correspond à un élément anatomique, par exemple, la vésicule biliaire (M4), le canal cystique (M5), l'artère cystique (M6), le triangle hépatocystique (M7) et la plaque cystique (M8) auxquels sont ajoutés les outils chirurgicaux (M9) et l'arrière-plan (M10). Pour un pixel de l'image I à traiter 35, les cellules des matrices correspondant à ce pixel indiqueront respectivement comme valeur 1 si le pixel représente une partie de ces éléments anatomiques (ou les outils et l'arrière-plan) et 0 si le pixel ne représente pas une partie de ces éléments anatomiques (ou les outils et l'arrière-plan).

La fonction f₂ associe respectivement au couple (I, f₁(I)) le triplet (c1, c2, c3) représentant la validation ou non de chaque critère prédéfini (Crit 1, Crit 2, Crit 3). Ainsi :
- c1 peut prendre la valeur « 1 » lorsque le triangle hépatocystique (plus précisément les éléments de traitement représentant le triangle hépatocystique sur l'image) ne comprend pas de tissus adipeux et fibreux (plus précisément, d'éléments de traitement représentant des tissus adipeux et fibreux) (Crit 1) et « 0 » sinon ;
- c2 peut prendre la valeur « 1 » lorsque le tiers inférieur de la vésicule biliaire est séparé du foie pour exposer la plaque cystique (Crit 2) et « 0 » sinon;
- c3 peut prendre la valeur « 1 » lorsqu'il apparait que le canal cystique et l'artère cystique pénètre dans la vésicule biliaire (Crit 3) et « 0 » sinon.

Les paramètres des deux fonctions paramétrées f₁ et f₂ sont déterminés, notamment en utilisant des algorithmes d'apprentissages automatiques, puis stockés sur la mémoire 43.

La première fonction paramétrée f₁ peut être définie comme un réseau de neurones artificiels d'une ou plusieurs couches de neurones. Chaque neurone d'une couche du réseau est une fonction d'activation qui a pour entrées des valeurs pondérées des sorties de la couche précédente. Les fonctions d'activation peuvent être, par exemple, des fonctions sigmoïdes, des fonctions tangentes hyperboliques, ou encore des fonctions de Heaviside. Les pondérations constituent les paramètres de la fonction f₁. Pour déterminer ces paramètres, un algorithme d'apprentissage automatique est utilisé avec des images de vidéos de référence (ou de séquences d'images de référence). Ainsi, l'algorithme d'apprentissage automatique détermine les paramètres afin de minimiser la distance entre les résultats de f₁ lorsque celle-ci est appliquée sur les images et les résultats attendus, à savoir, les valeurs attendues des cellules des matrices M4 à M10. Les valeurs attendues peuvent être déterminées par un spécialiste du domaine relatif à l'acte opératoire spécifique, par exemple, en déterminant sur chaque image utilisée (pour déterminée les paramètres) les différents éléments anatomiques.

De même que pour la première fonction paramétrée f₁, la deuxième fonction paramétrée f₂ peut être définie comme un réseau de neurones artificiels d'une ou plusieurs couches de neurones. Chaque neurone d'une couche du réseau est une fonction d'activation qui a pour entrées des valeurs pondérées des sorties de la couche précédente. Les fonctions d'activation peuvent être, par exemple, des fonctions sigmoïdes, des fonctions tangentes hyperboliques, ou encore des fonctions de Heaviside. Les pondérations constituent les paramètres de la fonction f₂. Pour déterminer ces paramètres, un algorithme d'apprentissage automatique est utilisé avec des images de vidéos de référence (ou de séquences d'images de référence). Ainsi, l'algorithme d'apprentissage automatique détermine les paramètres afin de minimiser la distance entre les résultats de la fonction f₂ lorsque celle-ci est appliquée sur les combinaisons de référence et les résultats attendus, à savoir, les valeurs c1, c2 et c3 précédemment indiquées. Ainsi, on minimise la distance entre les valeurs c1, c2 et c3 attendues et les résultats de la fonction f₂ lorsque celle-ci est appliquée sur des n-uplets de matrices (M1,..., M10) correspondant à des images de séquence d'images de référence utilisées pour réaliser l'apprentissage.

Une fois les paramètres des fonctions paramétrées f₁ et f₂ identifiés au moyen d'une base de données comportant des vidéos de références relatifs à l'acte opératoire spécifique, à savoir, ici une cholécystectomie, ceux-ci sont enregistrés dans la mémoire 43.

A l'étape S1, le dispositif de traitement du flux vidéo 40 reçoit, via le module d'interface avec la caméra chirurgicale 41, des données correspondant à l'encodage de la dernière image 35 de la séquence d'images 34 (ou flux vidéo). Par exemple, le dispositif de traitement du flux vidéo 40 reçoit des données dans un format MPEG correspondant à la dernière image 35. Les images de la séquence d'images 34 précédemment reçues sont en cours de traitement ou ont déjà été traitées par le dispositif de traitement du flux vidéo 40. La dernière image 35 est donc la prochaine image à traiter. Si le flux vidéo n'est pas interrompu, d'autres images seront reçues par le dispositif 40 et seront traitées à la suite de l'image 35.

A l'étape S2, le processeur 42 applique la fonction de traitement F sur l'image 35 pour obtenir le triplet de valeurs (c1, c2, c3) relatif aux trois critères considérés (Crit 1, Crit 2, Crit 3). L'image 35 peut être représentée par un triplet de matrices (M1, M2, M3) comme précédemment indiqué. Ainsi, le résultat de F sur ce triplet de matrice peut s'obtenir par F(M1, M2, M3) = f₂[(M1, M2, M3), f₁(M1, M2, M3)]= f₂(M1,..., M10).

La fonction de traitement F est avantageusement appliquée en une seule étape sur le triplet de matrices représentant l'image a traité 35 (M1, M2, M3), même si le traitement de l'image 35 peut aussi être réalisé en deux étapes, à savoir l'application de la première fonction paramétrée f₁ sur le triplet de matrices (M1, M2, M3) puis l'application de la deuxième fonction paramétrée f₂ sur la combinaison de l'image 35 (c'est-à-dire le triplet de matrice (M1, M2, M3)) et du résultat de la première fonction paramétrée f₁ (c'est-à-dire le 7-uplet de matrices (M4,..., M10)), c'est-à-dire l'application de la deuxième fonction paramétrée f₂ sur le n-uplet (M1,..., M10). Lorsque la fonction de traitement est appliquée en deux étapes, un contrôle optionnel peut être réalisé sur les résultats de la première fonction paramétrée f₁ afin de valider ou surveiller aisément la pertinence des résultats de la fonction de traitement F.

Ainsi, dans l'exemple de la figure 1, le résultat de la fonction de traitement F est (c1, c2, c3) = (1, 1, 1), c'est-à-dire que les trois critères sont vérifiés. Le résultat de la fonction de traitement F peut être un triplet de valeurs, chaque valeur indiquant un niveau de confiance dans la vérification du critère. Par exemple, si (c1, c2, c3) = (58, 32, 99) alors on considère que le critère 1 est vérifié avec une confiance de 58%, le critère 2 est vérifié avec une confiance de 32% et le critère 3 est vérifié avec une confiance de 99%.

A l'étape S3, le processeur 42 détermine un état d'avancement de l'étape opératoire associé à la dernière image 34 sur la base des critères vérifiés ou non pour cette image. Dans l'exemple de la figure 1, l'étape opératoire de l'acte opératoire spécifique est une étape préparatoire requise avant de pratiquer l'ablation définitive de la vésicule biliaire. Les trois critères prédéfinis (Crit 1, Crit 2, Crit 3) étant vérifiés, l'état d'avancement de l'étape préparatoire est final (ou encore, 100% de l'étape préparatoire a été réalisée) sur l'image 35 de la séquence d'images 34. Ainsi, l'étape préparatoire peut être considérée comme terminée. Si l'un des critères n'est pas rempli, alors l'état d'avancement de l'étape préparatoire est inachevé (ou encore 66% de l'étape préparatoire a été réalisée). Si deux critères ne sont pas remplis, alors l'état d'avancement de l'étape préparatoire est inachevé (ou encore, 33% de l'étape préparatoire a été réalisée). Ou encore, si aucun des critères n'est rempli, alors l'état d'avancement de l'étape préparatoire est initial (ou encore, 0% de l'étape préparatoire a été réalisée).

L'état d'avancement est associé à une image (ici l'image 35) de la séquence d'images 34. Ainsi, le processeur 42 peut avoir calculé pour chaque image de la séquence d'images 34 un état d'avancement.

A l'étape S4, le processeur 42 détermine sur la base de l'état d'avancement calculé à l'étape S4, un écart avec un protocole opératoire.

L'écart peut être un indicateur nécessitant le traitement de plusieurs images de la séquence d'images 34. En effet, alors que l'état d'avancement peut être un état à un instant t de la prise de l'image 35, l'écart peut être une analyse considérant l'enchainement des états d'avancement (c'est-à-dire l'enchainement de la validation des critères). Ainsi, le processeur 42 peut calculer l'évolution des états d'avancement de la séquence d'images 34 et en déduire un écart, par exemple :
- une différence d'ordre de validation des critères prédéfinis par rapport à l'ordre de validation des critères jusqu'au même état d'avancement du protocole ; ou/et
- une différence entre la partie de l'élément anatomique sur lequel agit le chirurgien et celle qui au vu du protocole devrait être concernée par les actions du chirurgien immédiatement après l'état d'avancement calculé à l'étape S3 ;
- une différence entre l'évolution de l'état d'avancement de la séquence d'images 34.

Cette étape est optionnelle.

A l'étape S5, le processeur 42 détermine des écarts entre des valeurs de caractéristiques d'un groupe d'éléments de traitement, c'est-à-dire un ensemble d'éléments de traitement représentant une partie d'élément anatomique et des valeurs de référence de caractéristiques (par exemple, des moyennes des caractéristiques des groupes d'éléments de traitement représentatifs de la même partie de l'élément anatomique) relatives au même état d'avancement que l'état d'avancement associé à l'image 35. Pour déterminer les écarts, des plages de valeurs peuvent être déterminées. Ainsi, les écarts correspondent au fait que des groupes de pixels présentent des caractéristiques (taille, forme, disposition ou encore couleur) dont les valeurs sortent des plages de valeurs comportant les valeurs de référence, c'est-à-dire des parties d'éléments anatomiques dont les caractéristiques ne sont pas normales.

A l'étape S6, le processeur 42 détermine les niveaux de risque relatifs à chaque écart déterminé à l'étape S5. Plusieurs niveaux de risques peuvent être prévus pour chaque caractéristique correspondant alors à plusieurs plages de valeurs imbriquées les unes dans les autres. Le niveau de risque est relatif à des risques liés à l'acte opératoire pratiqué.

Les étapes S5 et S6 font partie d'un mode de réalisation qui peut être combiné avec les autres modes de réalisation, par exemple celui décrit à l'étape S4. Ces étapes sont néanmoins optionnelles.

A l'étape S7, le processeur 42 contrôle le module d'interface avec le moyen d'affichage 44 afin d'afficher des informations sur le moyen d'affichage 50. Les informations à afficher sont des informations dépendantes de l'état d'avancement déterminé à l'étape S3. Le moyen d'affichage dans l'exemple de la figure 1 est un écran situé dans la salle du bloc opératoire et les informations affichées dessus sont destinées au chirurgien pratiquant l'acte opératoire. Toutefois, le moyen d'affichage peut être de toute autre nature (lunette, projecteur...) et peut être à destination d'autre personne que le chirurgien, par exemple, un membre administratif de la clinique ou de l'hôpital ou encore un chirurgien supervisant l'acte opératoire.

Les informations affichées peuvent être :
- une information dépendante de l'écart avec un protocole opératoire déterminé à l'étape S4 (qui peut être présentée sous forme d'alerte). Ainsi, l'écran peut afficher que les critères n'ont pas été validés dans l'ordre requis par le protocole. L'écran peut également afficher que l'élément anatomique sur lequel agit le chirurgien ne correspond pas à un élément anatomique qui au stade de l'état d'avancement est impliqué dans le cadre du protocole. Il peut également s'agir d'afficher une information indiquant que l'état d'avancement ne correspond pas à celui du protocole opératoire, par exemple, un état d'avancement évoluant très vite au début de l'étape opératoire considéré puis très lentement ;
- une information indiquant que l'un des critères testés à l'étape S2 n'est pas vérifié ;
- une information indiquant les critères vérifiés comme décrits à la figure 1 où une information 52 indique que les trois critères ont été vérifiés (c'est-à-dire validés) ;
- une information validant une étape d'une étape opératoire ou une information autorisant le démarrage d'une étape opératoire suivante. Dans l'exemple de la figure 1, suite à la vérification des trois critères, l'étape préparatoire est validée par un « OK » 51 ;
- une information sur le niveau de risque déterminé à l'étape S6 ;
- une zone indiquée sur l'image 35. Cette zone peut indiquer la zone où une action chirurgicale est à réaliser. A l'inverse, cette zone peut indiquer une zone où il n'y a pas d'action chirurgicale est à réaliser ou dans laquelle se trouve un élément anatomique à risque ;
- une information sur l'état d'avancement de l'étape opératoire déterminée à l'étape S3 ;
- des images d'une séquence d'images de référence (ou une vidéo de référence) relative à l'acte opératoire spécifique. Les images ou la vidéo étant des images correspondant à un l'état d'avancement identique ou postérieur à celui déterminé à l'étape S3.

L'affichage de l'information peut se faire en combinaison avec l'image 35 comme cela est le cas dans l'exemple de la figure 1. Cette information peut être adaptée à la criticité de l'étape opératoire en cours et de la criticité de l'information à afficher. Ainsi, le fait de valider les critères peut être affiché de manière plus discrète que l'information sur le niveau de risque ou encore la zone dans laquelle se trouve un élément anatomique à risque. Les informations affichées sur l'écran de la figure 1 sont affichées en haut à gauche de l'écran, toute autre disposition est possible.

Les étapes S2 à S7 peuvent être répétées tant que le module d'encodage 33 transmet des images à traiter.

A l'étape S8, le processeur 42 contrôle le dispositif pour stocker (ou stocker avec une définition plus grande et/ou en conservant plus d'images de cette partie du flux vidéo relativement au stockage du reste du flux vidéo) des images de la séquence comprise temporellement autour d'une image de la séquence d'images (par exemple, les images de la prochaine minute de vidéo qui suivent l'image 35) en fonction de la criticité l'étape opératoire relative à l'état d'avancement.

Le caractère critique ou non d'une étape opératoire peut être indiqué par le protocole opératoire.

Le stockage peut être effectué dans la mémoire 43 ou dans un centre de données. Ainsi, il est possible de conserver une plus grande quantité de flux vidéo en conservant en priorité les parties pertinentes de ces flux.

## Revendications

1. Un dispositif de traitement d'un flux vidéo relatif à un acte opératoire spécifique,
ledit dispositif comprenant :
une interface de réception de flux vidéo
un processeur, et
une mémoire stockant des instructions, de sorte que lorsque ces instructions sont exécutées par le processeur, elles configurent le dispositif pour:
- réceptionner via l'interface de réception de flux vidéo le flux vidéo comprenant une séquence d'images dont une image à traiter représentant au moins une partie d'un élément anatomique, ladite image à traiter étant formée par des éléments de traitement;
- déterminer au moyen d'une fonction de traitement si un critère est ou non vérifié sur l'image à traiter, la fonction de traitement étant composé d'une première fonction paramétrée et d'une deuxième fonction paramétrée :
• des paramètres de la première fonction paramétrée étant obtenus par un algorithme d'apprentissage automatique sur la base d'une image d'une séquence d'images de référence de sorte que le résultat de la première fonction paramétrée appliquée sur l'image permette de déterminer les éléments de traitement de l'image représentatifs de la partie de l'élément anatomique, la séquence d'images de référence étant précédemment enregistrée et relative à l'acte opératoire spécifique, l'image de la séquence d'images de référence représentant au moins une partie d'un élément anatomique,
• des paramètres de la deuxième fonction paramétrée étant obtenus par un algorithme d'apprentissage automatique sur la base de l'image de la séquence d'images de référence combinée à un résultat de la première fonction paramétrée appliquée sur l'image de la séquence d'images de référence de sorte que le résultat de la deuxième fonction paramétrée appliquée sur l'image combinée à un résultat de la première fonction paramétrée appliquée sur l'image permette de déterminer si le critère est ou non vérifié;
- déterminer un état d'avancement associé à l'image à traiter en fonction de si le critère est vérifié ou non, l'état d'avancement étant représentatif d'un état d'avancement d'une étape opératoire de l'acte opératoire spécifique.

2. Le dispositif selon l'une des revendications précédentes dans lequel lorsque les instructions sont exécutées par le processeur, elles configurent le dispositif pour:
- en fonction de l'état d'avancement déterminé, stocker des images de la séquence d'images comprises dans un intervalle temporel autour de l'image à traiter.

3. Le dispositif selon la revendication 2 dans lequel le nombre d'images stockées est fonction d'une criticité de l'étape opératoire et/ou de l'état d'avancement déterminé.

4. Le dispositif selon l'une des revendications précédentes comprenant en outre un moyen d'affichage et dans lequel lorsque les instructions sont exécutées par le processeur, elles configurent le dispositif pour:
- afficher avec l'image à traité, une information dépendante de l'état d'avancement sur le moyen d'affichage.

5. Le dispositif selon la revendication 4 dans lequel lorsque les instructions sont exécutées par le processeur, elles configurent le dispositif pour:
- déterminer un écart avec un protocole opératoire sur la base de l'état d'avancement;
dans lequel l'information dépendante de l'état d'avancement est dépendante de l'écart déterminé.

6. Le dispositif selon l'une des revendications 4 à 5 dans lequel l'affichage de l'information dépendante de l'état d'avancement est fonction d'une criticité de l'étape opératoire représentée par l'état d'avancement.

7. Le dispositif selon l'une des revendications 4 à 6 dans lequel l'information dépendante de l'état d'avancement comprend une information indiquant que le critère n'est pas validé.

8. Le dispositif selon l'une des revendications 4 à 7 dans lequel l'information dépendante de l'état d'avancement comprend une information validant une étape d'une étape opératoire ou une information autorisant le démarrage d'une étape opératoire suivante.

9. Le dispositif selon l'une des revendications 4 à 8 dans lequel lorsque les instructions sont exécutées par le processeur, elles configurent le dispositif pour:
- déterminer un écart entre au moins une valeur d'une caractéristique d'un groupe d'éléments de traitement de l'image représentatifs de la partie de l'élément anatomique et une valeur de référence, la valeur de référence étant une moyenne de la valeur de la caractéristique de groupes d'éléments de traitement représentatifs de la partie de l'élément anatomique et relatif au même état d'avancement que l'état d'avancement;
- déterminer un niveau de risque lié à cet écart ;
dans lequel l'information dépendante de l'état d'avancement comprend le niveau de risque.

10. Le dispositif selon l'une des revendications 4 à 9 dans lequel l'information dépendante de l'état d'avancement comprend :
- une zone dans laquelle une action chirurgicale est à réaliser ; et/ou
- une zone dans laquelle il n'y a pas d'action chirurgicale ; et/ou
- une zone dans laquelle se trouve un élément anatomique requérant une attention particulière ; et/ou
- une zone correspondant à des éléments de traitement considérés pour déterminer l'état d'avancement.

11. Le dispositif selon l'une des revendications 4 à 10 dans lequel l'information dépendante de l'état d'avancement comprend des images de la séquence d'images de référence en commençant par l'image correspondant à l'état d'avancement.

12. Un programme informatique comportant des instructions qui lorsque celles-ci sont exécutées par un processeur mettent en œuvre un procédé de traitement d'un flux vidéo relatif à un acte opératoire spécifique comprenant :
- la réception via l'interface de réception de flux vidéo du flux vidéo comprenant une séquence d'images dont une image à traiter représentant au moins une partie d'un élément anatomique, ladite image à traiter étant formée par des éléments de traitement;
- la détermination au moyen d'une fonction de traitement de si un critère est ou non vérifié sur l'image à traiter, la fonction de traitement étant composé d'une première fonction paramétrée et d'une deuxième fonction paramétrée :
• des paramètres de la première fonction paramétrée étant obtenus par un algorithme d'apprentissage automatique sur la base d'une image d'une séquence d'images de référence de sorte que le résultat de la première fonction paramétrée appliquée sur l'image permette de déterminer les éléments de traitement de l'image représentatifs de la partie de l'élément anatomique, la séquence d'images de référence étant précédemment enregistrée et relative à l'acte opératoire spécifique, l'image de la séquence d'images de référence représentant au moins une partie d'un élément anatomique,
• des paramètres de la deuxième fonction paramétrée étant obtenus par un algorithme d'apprentissage automatique sur la base de l'image de la séquence d'images de référence combinée à un résultat de la première fonction paramétrée appliquée sur l'image de la séquence d'images de référence de sorte que le résultat de la deuxième fonction paramétrée appliquée sur l'image combinée à un résultat de la première fonction paramétrée appliquée sur l'image permette de déterminer si le critère est ou non vérifié;
- la détermination d'un état d'avancement associé à l'image à traité en fonction de si le critère est vérifié ou non, l'état d'avancement étant représentatif d'un état d'avancement d'une étape opératoire de l'acte opératoire spécifique.

## Patentansprüche

1. Vorrichtung zur Verarbeitung eines Videostroms, der sich auf einen spezifischen operativen Eingriff bezieht, wobei die Vorrichtung umfasst:
eine Schnittstelle zum Empfang des Videostroms,
einen Prozessor und
einen Speicher, der Befehle speichert, sodass diese Befehle, wenn sie vom Prozessor ausgeführt werden, die Vorrichtung dazu konfigurieren:
- den Videostrom über die Schnittstelle zum Empfang von Videoströmen zu empfangen, wobei der Videostrom eine Bildsequenz umfasst, darunter ein zu verarbeitendes Bild, das wenigstens einen Teil eines anatomischen Elements darstellt, wobei das zu verarbeitende Bild aus Verarbeitungselementen gebildet ist;
- mittels einer Verarbeitungsfunktion zu bestimmen, ob ein Kriterium für das zu verarbeitende Bild erfüllt ist oder nicht, wobei die Verarbeitungsfunktion aus einer ersten parametrierten Funktion und einer zweiten parametrierten Funktion besteht:
• wobei die Parameter der ersten parametrierten Funktion durch einen Algorithmus des maschinellen Lernens auf der Grundlage eines Bildes einer Referenzbildsequenz erhalten werden, so dass das Ergebnis der ersten parametrierten Funktion, die auf das Bild angewendet wird, es ermöglicht, die Verarbeitungselemente des Bildes zu bestimmen, die für den Teil des anatomischen Elements repräsentativ sind, wobei die Referenzbildsequenz zuvor aufgezeichnet wurde und sich auf den spezifischen operativen Eingriff bezieht, wobei das Bild der Referenzbildsequenz wenigstens einen Teil eines anatomischen Elements darstellt,
• wobei die Parameter der zweiten parametrierten Funktion durch einen Algorithmus des maschinellen Lernens auf der Grundlage des Bildes der Referenzbildsequenz in Kombination mit einem Ergebnis der ersten parametrierten Funktion, die auf das Bild der Referenzbildsequenz angewendet wird, erhalten werden, so dass das Ergebnis der zweiten parametrierten Funktion, die auf das Bild angewendet wird, in Kombination mit einem Ergebnis der ersten parametrierten Funktion, die auf das Bild angewendet wird, es ermöglicht, zu bestimmen, ob das Kriterium erfüllt ist oder nicht;
- einen mit dem zu verarbeitenden Bild verbundenen Fortschrittszustands in Abhängigkeit davon zu bestimmen, ob das Kriterium erfüllt ist oder nicht, wobei der Fortschrittszustand einen Fortschrittszustand eines Operationsschritts des spezifischen operativen Eingriffs darstellt.

2. Vorrichtung nach einem der vorhergehenden Ansprüche, wobei die Befehle, wenn sie vom Prozessor ausgeführt werden, die Vorrichtung dazu konfigurieren:
- in Abhängigkeit vom ermittelten Fortschrittszustand Bilder der Bildsequenz zu speichern, die in einem Zeitintervall um das zu verarbeitende Bild herum enthalten sind.

3. Vorrichtung nach Anspruch 2, wobei die Anzahl der gespeicherten Bilder von der Kritikalität des Operationsschritts und/oder dem ermittelten Fortschrittszustand abhängt.

4. Vorrichtung nach einem der vorstehenden Ansprüche, die ferner ein Anzeigemittel umfasst, und wobei die vom Prozessor ausgeführten Befehle die Vorrichtung dazu konfigurieren:
- zusammen mit dem zu verarbeitenden Bild eine vom Fortschrittszustand abhängige Information auf dem Anzeigemittel anzuzeigen.

5. Vorrichtung nach Anspruch 4, wobei die Befehle, wenn sie vom Prozessor ausgeführt werden, die Vorrichtung dazu konfigurieren:
- auf der Grundlage des Fortschrittszustands eine Abweichung von einem Operationsprotokoll zu ermitteln; wobei die vom Fortschrittszustand abhängige Information von der ermittelten Abweichung abhängt.

6. Vorrichtung nach einem der Ansprüche 4 bis 5, wobei die Anzeige der vom Fortschrittszustand abhängigen Informationen von der Kritikalität des durch den Fortschrittszustand dargestellten Operationsschritts abhängt.

7. Vorrichtung nach einem der Ansprüche 4 bis 6, wobei die vom Fortschrittszustand abhängige Information eine Information umfasst, die angibt, dass das Kriterium nicht validiert ist.

8. Vorrichtung nach einem der Ansprüche 4 bis 7, wobei die vom Fortschrittszustand abhängige Information eine Information umfasst, die einen Schritt eines Operationsschritts validiert, oder eine Information, die den Start eines folgenden Operationsschritts autorisiert.

9. Vorrichtung nach einem der Ansprüche 4 bis 8, wobei die Befehle, wenn sie vom Prozessor ausgeführt werden, die Vorrichtung dazu konfigurieren:
- eine Abweichung zwischen wenigstens einem Wert einer Eigenschaft einer Gruppe von Bildverarbeitungselementen, die den Teil des anatomischen Elements repräsentieren, und einem Referenzwert zu bestimmen, wobei der Referenzwert ein Mittelwert des Werts des Merkmals von Gruppen von Verarbeitungselementen ist, die den Teil des anatomischen Elements repräsentieren und sich auf denselben Fortschrittszustand wie der Fortschrittszustand beziehen;
- ein mit dieser Abweichung verbundenes Risikoniveau zu bestimmen,
wobei die vom Fortschrittszustand abhängige Information das Risikoniveau umfasst.

10. Vorrichtung nach einem der Ansprüche 4 bis 9, wobei die vom Fortschrittszustand abhängige Information umfasst:
- einen Bereich, in dem ein chirurgischer Eingriff durchgeführt werden soll; und/oder
- einen Bereich, in dem kein chirurgischer Eingriff erfolgt; und/oder
- einen Bereich, in dem sich ein anatomisches Element befindet, das besondere Aufmerksamkeit erfordert; und/oder
- einen Bereich, der den zur Bestimmung des Fortschrittszustands berücksichtigten Behandlungselementen entspricht.

11. Vorrichtung nach einem der Ansprüche 4 bis 10, wobei die vom Fortschrittszustand abhängige Information Bilder der Referenzbildsequenz umfasst, beginnend mit dem Bild, das dem Fortschrittszustand entspricht.

12. Ein Computerprogramm, umfassend Befehle, die, wenn sie von einem Prozessor ausgeführt werden, ein Verfahren zur Verarbeitung eines Videostroms in Bezug auf einen spezifischen operativen Eingriff umsetzen, umfassend:
- Empfangen des Videostroms über die Videostrom-Empfangsschnittstelle, wobei der Videostrom eine Bildsequenz umfasst, darunter ein zu verarbeitendes Bild, das wenigstens einen Teil eines anatomischen Elements darstellt, wobei das zu verarbeitende Bild aus Verarbeitungselementen gebildet wird;
- Bestimmung mittels einer Verarbeitungsfunktion, ob ein Kriterium auf dem zu verarbeitenden Bild erfüllt ist oder nicht, wobei die Verarbeitungsfunktion durch eine erste parametrierte Funktion und eine zweite parametrierte Funktion vorgegeben ist:
• wobei die Parameter der ersten parametrierten Funktion durch einen Algorithmus des maschinellen Lernens auf der Grundlage eines Bildes einer Referenzbildsequenz erhalten werden, so dass das Ergebnis der auf das Bild angewendeten ersten parametrierten Funktion es ermöglicht, die Verarbeitungselemente des Bildes zu bestimmen, die für den Teil des anatomischen Elements repräsentativ sind, wobei die Referenzbildsequenz zuvor aufgezeichnet wurde und sich auf den spezifischen operativen Eingriff bezieht, wobei das Bild der Referenzbildsequenz wenigstens einen Teil eines anatomischen Elements darstellt,
• wobei die Parameter der zweiten parametrierten Funktion durch einen Algorithmus des maschinellen Lernens auf der Grundlage des Bildes der Referenzbildsequenz in Kombination mit einem Ergebnis der ersten parametrierten Funktion, die auf das Bild der Referenzbildsequenz angewendet wird, ermittelt werden, so dass das Ergebnis der zweiten parametrierten Funktion, die auf das Bild angewendet wird, in Kombination mit einem Ergebnis der ersten parametrierten Funktion, die auf das Bild angewendet wird, es ermöglicht, zu bestimmen, ob das Kriterium erfüllt ist oder nicht;
- die Bestimmung eines mit dem zu verarbeitenden Bild verbundenen Fortschrittszustands in Abhängigkeit davon, ob das Kriterium erfüllt ist oder nicht, wobei der Fortschrittszustand einen Fortschrittszustand eines Arbeitsschritts des spezifischen Arbeitsschritts darstellt

## Claims

1. Device for processing a video stream related to a specific surgical procedure, said device comprising:
a video-stream receiving interface
a processor, and
a memory storing instructions, so that, when these instructions are executed by the processor, they configure the device to:
- receive through the video-stream reception interface a video stream comprising a sequence of images, one of which to be processed shows at least a part of an anatomical element, said image to be processed being formed by processing elements;
- determine, by means of a processing function, whether a criterion is met or not on the image to be processed, the processing function consisting of a first parameterised function and a second parameterised function:
• parameters of the first parameterised function being obtained by a machine learning algorithm based on an image from a sequence of reference images, such that the result of the first parameterised function applied to the image makes it possible to determine the image-processing elements that are representative of the part of the anatomical element, the sequence of reference images having been previously recorded and pertaining to the specific surgical procedure, with the image from the sequence of reference images representing at least a part of an anatomical element,
• parameters of the second parameterized function being obtained by a machine learning algorithm based on the image of the sequence of reference images combined with a result of the first parameterised function applied to the image of the sequence of reference images such that the result of the second parameterised function applied to the image combined with a result of the first parameterised function applied to the image makes it possible to determine whether the criterion is met or not;
- determine a state of progress associated with the image to be processed according to whether the criterion is met or not, with the state of progress representing a state of progress of an operating step of the specific surgical procedure.

2. Device according to one of the preceding claims, wherein, when the instructions are executed by the processor, they configure the device to:
- depending on the determined state of progress, store images from the sequence of images included within a time interval around the image to be processed.

3. Device according to claim 2, wherein the number of stored images is dependent on the criticality of the operating step and/or the determined state of progress.

4. Device according to one of the preceding claims, further comprising a display means and wherein, when the instructions are executed by the processor, they configure the device to:
- display, with the image to be processed, information dependent on the state of progress on the display means.

5. Device according to claim 4, wherein, when the instructions are executed by the processor, they configure the device to:
- determine a discrepancy with an operating protocol based on the state of progress;
wherein the information dependent on the state of progress is dependent on the discrepancy determined.

6. Device according to one of claims 4 to 5, wherein the display of information dependent on the state of progress is dependent on the criticality of the operating step represented by the state of progress.

7. Device according to one of claims 4 to 6, wherein the information dependent on the state of progress comprises information indicating that the criterion has not been validated.

8. Device according to one of claims 4 to 7, wherein the information dependent on the state of progress comprises information validating a step of an operating step or information authorising the commencement of a following operating step.

9. Device according to one of claims 4 to 8, wherein, when the instructions are executed by the processor, they configure the device to:
- determine a discrepancy between at least one value of a characteristic of a group of image processing elements that are representative of the anatomical element and a reference value, the reference value being a mean of the characteristic values of groups of processing elements representative of the anatomical element and relating to the same stage of progress as the state of progress;
- determine a level of risk relating to this discrepancy;
wherein the information dependent on the state of progress comprises the level of risk.

10. Device according to one of claims 4 to 9, wherein the information dependent on the state of progress comprises:
- an area in which a surgical action is to be performed; and/or
- an area in which there is no surgical action; and/or
- an area in which an anatomical element requiring particular attention is located; and/or
- an area corresponding to processing elements considered to determine the state of progress.

11. Device according to one of claims 4 to 10, wherein the information dependent on the state of progress comprises images from the sequence of reference images starting with the image that corresponds to the state of progress.

12. Computer program including instructions that, when they are executed by a processor, implement a method for processing a video stream related to a specific surgical procedure, comprising:
- receiving through the video-stream reception interface a video stream comprising a sequence of images, one of which to be processed represents at least a part of an anatomical element, said image to be processed being formed by processing elements;
- determining by means of a processing function whether or not a criterion is met on the image to be processed, the processing function being composed of a first parameterised function and a second parameterised function:
• parameters of the first parameterised function being obtained by a machine learning algorithm based on an image from a sequence of reference images, such that the result of the first parameterised function applied to the image makes it possible to determine the image-processing elements that are representative of the part of the anatomical element, the sequence of reference images having been previously recorded and pertaining to the specific surgical procedure, with the image from the sequence of reference images representing at least a part of an anatomical element,
• parameters of the second parameterised function being obtained by a machine learning algorithm based on the image of the sequence of reference images combined with a result of the first parameterised function applied to the image of the sequence of reference images such that the result of the second parameterised function applied to the image combined with a result of the first parameterised function applied to the image makes it possible to determine whether the criterion is met or not;
- determining a state of progress associated with the image to be processed according to whether the criterion is met or not, the state of progress being representative of a state of progress of an operating step of the specific surgical procedure.
